# EUROPEAN PATENT SPECIFICATION

(11) **EP 0 538 276 B1**
(45) Date of publication and mention of the grant of the patent: **27.12.1996**
(21) Application number: 91910974.4
(22) Date of filing: 11.06.1991
(51) Int. Cl.: A61F 2/30

(54) **METHOD OF PROVIDING A PROSTHETIC DEVICE**
VERFAHREN ZUM BEREITSTELLEN EINER PROTHETISCHEN VORRICHTUNG
PROCEDE DE MISE A DISPOSITION D'UN DISPOSITIF PROTHETIQUE

(30) Priority: 25.06.1990 GB 9014057
(43) Date of publication of application: 28.04.1993
(73) Proprietor: DEPUY INTERNATIONAL LIMITED, Beeston, Leeds LS11 8DT (GB)
(72) Inventor: ELLOY, Martin, Depuy Internat. Limited, Beeston, Leeds LS11 8DT (GB)
(74) Representative: Orr, William McLean
(86) International application number: GB9100936
(87) International publication number: WO9200045

(56) References cited:
- EP-A- 0 149 527
- EP-A- 0 163 121
- EP-A- 0 273 871
- EP-A- 0 366 945
- FR-A- 2 577 697
- LE NOUVEL AUTOMATISME, vol. 30, no. 55, June 1985, (Paris, FR), J.-C. MORETTON et al.: "Problèmes de prothèse, problèmes de C.F.A.O.", pages 50-54, see page 52, column 1, line 1 - column 2, line 8; figure 2; page 53, figure
- BIOMEDIZINISCHE TECHNIK, vol. 33, no. 6, June 1988, (Berlin, DE), "Mit dem Computer zum massgeschneiderten Hüftgelenk", pages 153,154, see page 154, column 1, line 22 - column 2, line 28

## Description

This invention relates to a method of manufacture of a prosthetic implant of a form to suit the particular requirements of a patient.

The invention has been developed primarily in connection with the manufacture of hip implants for human use, but it should be understood that the invention has general application to the manufacture of prosthetic implants for human or animal use and including, inter alia, knee implants.

Conventionally, hip implants are made and supplied in either one of two ways. In a first technique, which is a pre-operative technique, a range of standard forms of implants e.g. different shapes and/or sizes, are manufactured and made available to surgeons, and it is then necessary to carry out necessary surgery to form a cavity of a suitable form to receive the selected form of implant. Thus, the femoral cavity will be prepared with approximately matching geometry to the implant, using a variety of different tools, including femoral rasps or broaches. One particularly advantageous recent improvement to a femoral rasp is disclosed in more detail in WO-A-90/03764.

In a second technique, pre-formed standard shapes/sizes of implants can be machined down to size to suit a pre-prepared femoral cavity in the manner disclosed in more detail in Stewal EP-A-0129531.

A disadvantage of the first technique is that it is limited in its ability to match ideally a limited range of pre-manufactured implants to the infinite range of anatomical geometry. A disadvantage of the second technique is that it is limited by the expense and inconvenience of providing the necessary plant and equipment close to the operating theatre as well as the evident risks associated with the short time available in which to carry out the manufacture, and also quality assurance problems. Thus, the implant has to be machined down to size following formation of a model from the cavity formed in the patient.

US-4658808 which is a patent family member of EP-A-0163121 discloses an arrangement for preparing an anatomically measured endoprosthesis using radiographs, a set of a limited number of prosthesis templates, and a set of corresponding prosthesis models. The templates are used to identify an appropriate configuration for the prosthesis, from which a trial prosthesis can be prepared.

The present invention seeks to provide an improved method of providing a prosthetic implant, the design of which can be derived accurately from patient parameters and without the necessity for pre-operative surgery to be carried out to set such parameters.

Accordingly, the invention provides a method of providing a prosthetic device for implantation in a bone cavity of a patient, which comprises:
(a) preparing an image of the bone cavity,
(b) providing a plurality of sets of templates of a general shape appropriate to the implant to be fitted in the bone cavity, the templates of each set comprising a range of progressively varying shapes or sizes or both,
(c) making a selection from the said sets of templates and comparing the selected template with the image to determine whether the template provides a suitable match with the profile of the cavity and, if necessary, making an alternative selection, and
(d) manufacturing an implant with a configuration corresponding to the selected template, or selecting an implant from a plurality of already-manufactured implants, with a configuration corresponding to the selected template,
characterised in that the templates form a two-dimensional model of the required implant which comprises a central core, a proximo-medial segment, a proximo-lateral segment and a proximo-anterior segment.

Preferably, the comparison takes place by arranging the image and the template one upon the other and conveniently the template is transparent, so that it can be positioned over the image. However, this is not essential to the invention, as evidently a transparent image sheet could be placed over a template (which may or may not be transparent) so that visual comparison can be made between the outline of the image and the outline of the preferred implant shape defined by the template.

The template may have the required shape of an implant applied thereon by the surgeon who will be carrying out the implantation of the prosthetic device, and this may be carried out by supplying template sheets having reference lines thereon, and which can be lined-up with corresponding references on the image, and the surgeon may then draw, or use any other suitable means e.g. an electronic pen, to mark his own selection of the appropriate outline for the implant.

The surgeon may then compare his own selected outline closely with the image, and make any correction which he thinks advisable e.g. by erasing part of the previous outline, or by applying a fresh template with a new outline marked on it. The surgeon then passes the template to the manufacturer / supplier of implants, who then preferably converts the information into digital form, from which is derived outline elevational views of the implant, and preferably also a three dimensional illustration, all of which are then supplied to the surgeon for final approval before the required implant is taken from stock, or manufactured to order.

In the case of a hip prosthesis manufacture, the images of the bone cavity will usually be A / P (anterior / posterior) and lateral views, and the template will have reference lines corresponding with each view, so that the surgeon can mark out his preferred outline shape of the required implant for both views.

The particular advantage of the above described techniques used in the manufacture of a prosthetic implant to suit a particular form of bone cavity of a patient is that design freedom is given to the surgeon who will be carrying out the implantation, and from his own experience he can select what appears to him to be the optimum profile.

The manufacturer then converts the data which is inputted by the surgeon into visual representations of the implant e.g. two elevational views and a three dimensional view, for return to the surgeon for final approval, before any supply or new manufacture takes place. This places the responsibility for the design shape on the person best qualified to make a suitable judgement for a particular patient, and yet provides a simple system which is easily carried out by the surgeon without any need to have special drawing skills. or special knowledge of drawing office techniques.

Finally, the responsibility for the design is entirely in the hands of the surgeon, and there can be no argument at a later date that the surgeon's design instructions have not been faithfully carried out as he intended.

The use of templates of the above described type comprise one preferred example for use in the invention. However, there are other ways in which a template can be provided which defines the required shape of an implant and which still relies upon input judgement made by the surgeon, but which involves exercise only of simple skills in order to build-up the required outline.

Therefore, in accordance with the invention, a plurality of sets of templates is provided of a general shape appropriate to the implant to be fitted in the bone cavity, and then a selection is made from the said sets and the selected template or templates are compared with the image outline to see whether they constitute a suitable match to the profile of the bone cavity, and if necessary an alternative selection is made.

This is then followed by manufacture of an implant of a form corresponding with the selected template, or selection of an implant from a preformed range of implants corresponding with the range of templates and of a form corresponding with the selected template.

By carrying out the method of the invention, an implant can be supplied which can correspond with the judgement of the surgeon as to the most suitable shape and/or size of template used with the image of the bone cavity, and without any preoperative surgery being required as is the case in the second known technique referred to earlier.

Preferably, the central core is built up from two portions, comprising a distal shaft to be located over the main tubular portion of the cavity, and a proximal portion to be located in the mouth or proximal end of the cavity.

The template in each set can be supplied in any required variation from one to another e.g. length, diameter, cross-section, and therefore the surgeon, using his skill and judgement and also knowledge of the needs of a particular patient, can then make a selection of the templates which he fits over the image. This information is conveyed to the supplier, who can then readily manufacture a matching implant, or else supply from stock.

The surgeon then can carry out necessary surgery and pre-formation of the bone cavity, once the implant has been delivered, and then can carry out implantation.

To facilitate accurate pre-preparation of the cavity to receive the selected form of implant, the supplier may also provide a range of forming tools of a form to suit the templates, or be able readily to manufacture forming tools to suit the selected model built up from the templates. This enables a suitable tool profile to be selected, which can adjust the shape of the bone cavity during necessary pre-preparation work, in order to receive the particular form of implant.

A forming tool may be built up from an assembly of rasp components, comprising a core component and one or more additional components slideably mounted on the core and selected to suit the model form. This rasp may be built up in the same way as the femoral rasp can be built up as disclosed in more detail in WO-A-90/03764.

Conveniently, the image of the bone cavity is formed by X-ray scanning, and in the case of a hip implant, the images will usually be the anterior/posterior (A/P) view and the lateral view. The surgeon will then usually place the two selected core components over the bone cavity of the A/P view, and then transfer this selection to the lateral view and make any necessary different selection (which may on occasions be a smaller size). The proximo-anterior segment will then be selected from its range and placed over the lateral view, and then the three components will be returned to the A/P view for selection and placement of the medial and proximo-lateral segments.

In the application of the invention to hip implants, preferably the proximal core portion has reference lines which will correspond to the lesser trochanter and the greater trochanter on the patient image.

The invention will now be described in more detail by way of example only, with reference to the accompanying drawings, in which:
Figure 1 is an X-ray image of an anterior/posterior view of a femoral component of a patient requiring a hip implant, and with one example of a two dimensional model of a required form of implant placed over the image;
Figure 2 is a lateral view of the femoral component with the model transferred thereto;
Figure 3 is a schematic view of a range of templates to form a distal core portion of the model;
Figures 4a and 4b are A/P and lateral views respectively of a proximal core portion of a range which can be used to build up the selected model form;
Figure 5 is a schematic illustration of one of a range of proximal inserts to be used in order to build-up the required model form:
Figure 6 shows schematically three proximal insert components to be used in building-up the required model form:
Figure 7 is a horizontal section illustrating the proximal end of the model built up from three selected components of the three separate insert components shown in figure 6, and fitted around the upper end of the proximal core portion shown in figures 4a and 4b;
Figures 8a and 8b are schematic illustrations of an instruction to the supplier from a surgeon for an implant to be supplied in a form matching that of the selected two dimensional model form built-up by the surgeon and fitting over the X-ray images of the bone cavity;
Figures 9a and 9b are respectively anterior / posterior and lateral views of an image of a femoral bone cavity of a patient into which a prosthetic device is to be implanted;
Figure 10 is a plan view of a further example of template for use with the views of Figures 9a and 9b;
Figure 11 is a plan view of the template of Figure 10 with an applied outline marking of a suggested profile of an implant to be used in the femoral bone cavity of the patient;
Figure 12 shows elevational views of an implant derived from data extracted from the marked template of Figure 11, and after suitable magnification to correspond with the magnification of the X-ray images;
Figure 13 is a three dimensional illustration of the implant corresponding with Figure 12 and obtained from computer generated graphics derived from the data extracted from Figure 12;
Figure 14 shows a first stage of converting a two dimensional image to three dimensional form;
Figure 15 shows a further stage;
Figure 16 is a section taken on A-A in Figure 15; and,
Figure 17 is a flow diagram showing the successive stages involved in the supply of a prosthetic implant of a design derived from data supplied by the surgeon involved in the implantation.

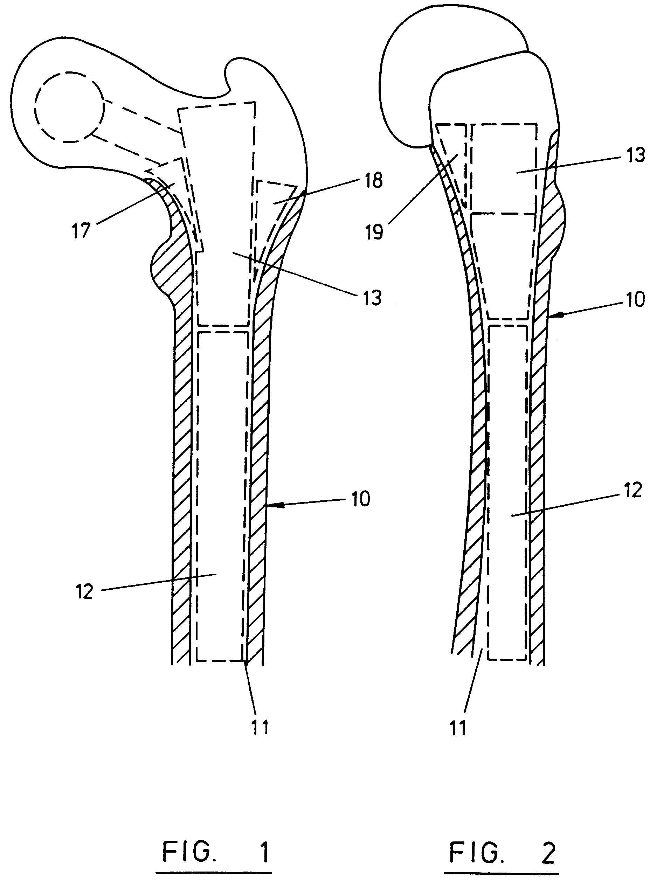

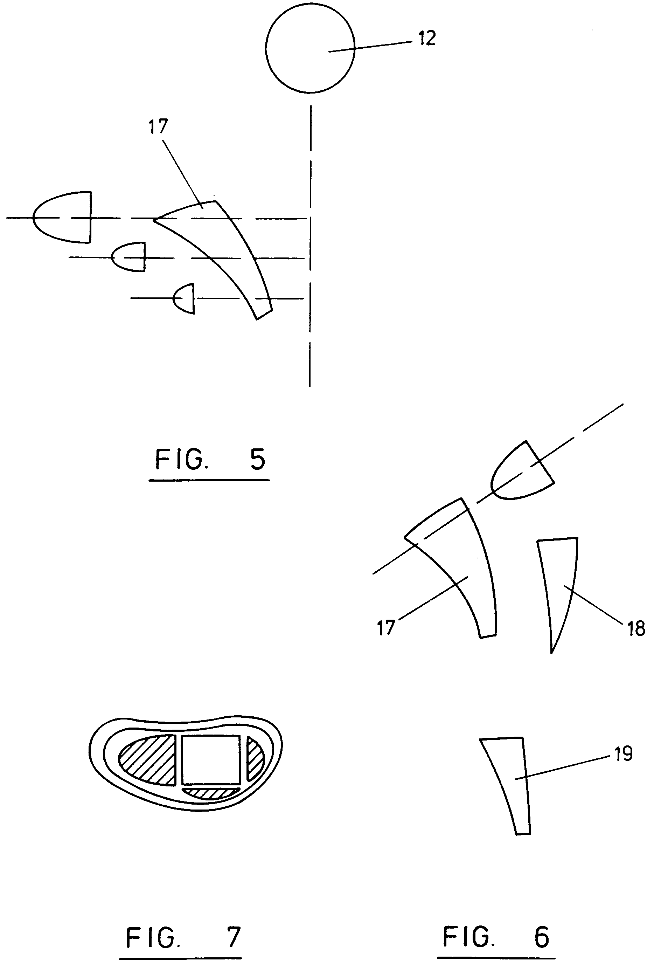

Referring first to Figures 1 to 8 of the drawings, there will be disclosed in detail one example of a method of manufacturing a prosthetic implant of a form to suit the particular requirements of a patient. This example will be described in connection with the manufacture of a replacement hip joint for a human patient, but it should be understood that this is by way of example only, and that the features of the invention may be applied generally in the manufacture and supply of prosthetic implants of other types, such as knee implants and for human or animal use.

Figures 1 and 2 are A / P and lateral views of X-ray images of a proximal femur 10 having a natural bone cavity 11, which is intended to receive a prosthetic implant. The two X-ray images prepared of the bone cavity appear on a transparent film, which can be placed on a suitable support surface, so that templates can be placed thereon in order to build up a two dimensional model of a required implant form.

Figures 1 and 2 are A/P and lateral views of a left femur, and show how a set of templates can be laid thereon to build up the required model form.

In the illustrated arrangement, five different template components are provided, and comprising first and second core portions 12 and 13, comprising the model form of the distal portion of the implant, and which usually is in the form of a circular cross-section rod.

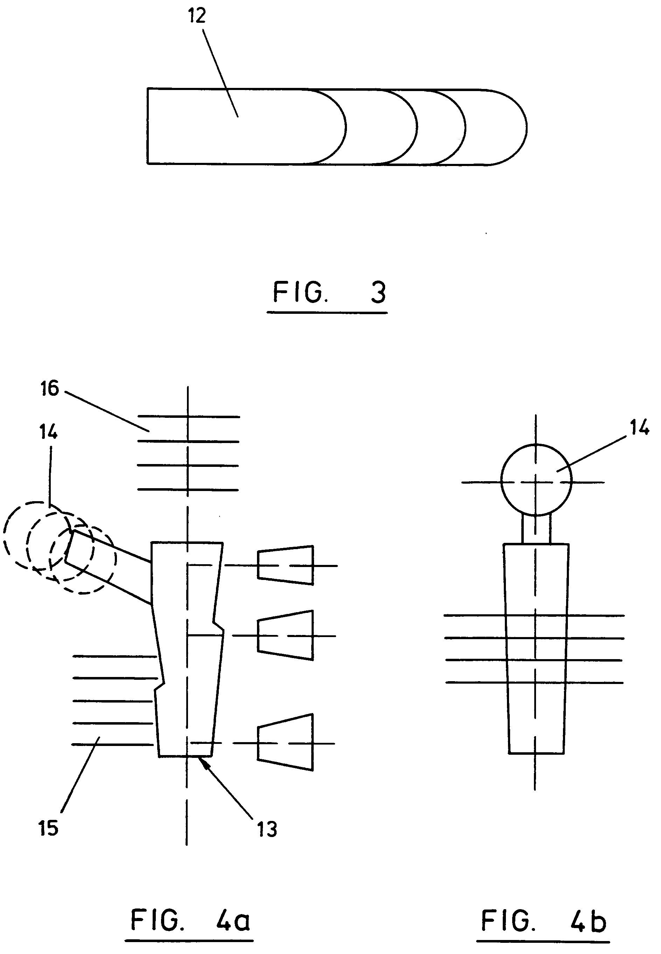

The portion 12 will be supplied in a range of sizes e.g. in varying length and/or diameter. as shown in figure 3, and the portion 13 also will be provided in a range of sizes and of varying cross section, and which is shown in figures 4a and 4b. The lateral extension of the simulated head 14 from the portion 13 also may be varied within a range of sizes. Also, reference lines 15 and 16 may be provided to enable the portion 13 to be lined up with the lesser trochanter and the greater trochanter respectively on the X-ray images of the bone cavity profile.

The further components of the sets of template comprise medial component 17 and lateral component 18 shown in figure 1, and anterior component 19 shown in figure 2.

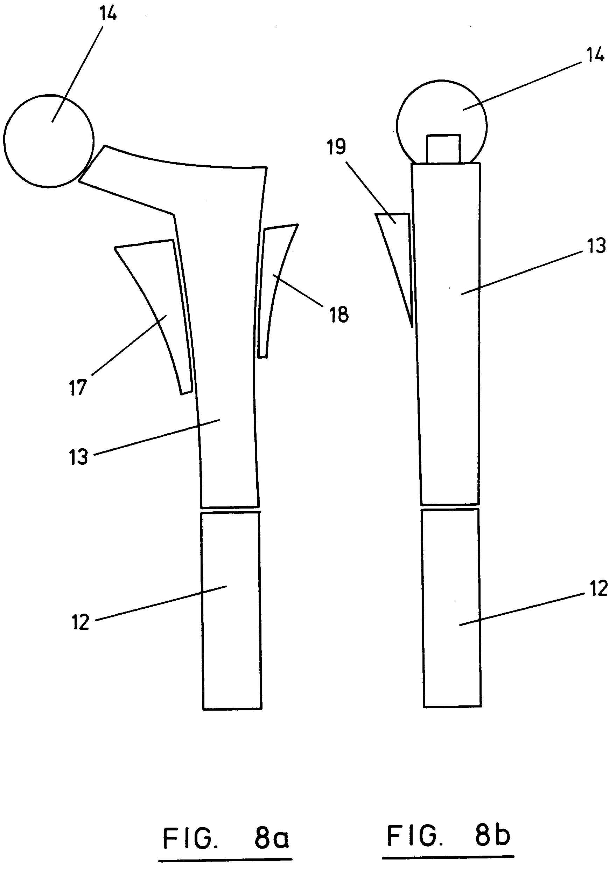

Usually, the surgeon will place suitably selected core portions 12 and 13 initially over the A/P view, and then transfer this selection to the lateral view and if necessary make an alternative selection, (if required usually a smaller size). The surgeon would then apply a selected anterior component 19 to fit over the lateral view, and then return these three components to the A/P view for application of selected medial component 17 and lateral component 18. The surgeon will use his skill and judgement as well as his knowledge of the needs of the patient, in determining the appropriate profile of each model segment from the ranges which will be made available to him. Once the surgeon is satisfied with his selection he will then complete an instruction to the manufacturer/supplier, in the form of a pro-forma order instruction as shown in figures 8a and 8b, giving his selection of the five components of the model, with suitable identifying characters. Upon receipt, a matching form of implant can be supplied from stock or else manufactured from data derived previously from the model segments.

Accordingly in its most general sense the invention provides a method of manufacture of a prosthetic implant of a form to fit a particular bone cavity of a patient and which comprises preparing an image of the bone cavity, providing at least one range of templates of a general shape appropriate to the implant to be fitted in the bone cavity, making a selection from this range of template and placing the selected template on the image to determine whether it constitutes a suitable match to the profile of the bone cavity, and if necessary making an alternative selection(s), and manufacturing an implant of a form corresponding with the selected template, or making a selection of an implant from a pre-formed range of implants corresponding with the range of templates and of a form corresponding with the selected template.

However, in the illustrated embodiment, five sets of templates are provided, with each set having a suitable range of components, and the image provided is a pair of X-ray images in the A/P view and the lateral view.

In addition, to facilitate accurate pre-preparation of the cavity to receive the selected implant form, the supplier may provide a range of forming tools of a form to suit the built-up model, or has stored data corresponding to the selected model to enable a forming tool to be built up to match this model, whereby a suitable tool profile can be selected which can adjust the shape of the bone cavity where necessary in order to receive the implant.

The forming tool may be built-up from an assembly of rasp components, comprising a core component, and one or more additional component slideably mounted thereon, in a manner disclosed in more detail in WO-A-90/03764. Alternatively, the tool may be constructed as a one-off construction.

Conveniently, the images of the bone cavity are formed by X-ray scanning. but the invention is not restricted to such means for providing the images.

Thus, in the practical application of the invention, an analysis of data will be obtained from CT scans and intra-operative moulds of femoral cavities, so that acceptable shape and size characteristics of prepared cavities and implants can be determined. These cavities are then divided into parts comprising the central core (corresponding to portions 12 and 13), a proximo-medial segment (17) a proximo-lateral segment (18) and a proximo-anterior segment (19). A range of different shapes and sizes of the segments will be determined.

Radiographic templates of all the templates of the segments, with appropriate magnification, will be provided to the surgeon who can assemble them in an "Identikit Fashion" to produce anterio-posterio and medio-lateral profiles considered best suited to the individual case. A forming tool in a form of a broach can be assembled from individual pre-manufactures modules which are slideably mounted thereon. The composite geometry is suitably processed and fed to a CNC machine to manufacture a matching implant from a range of suitable blanks and precuts. The assembled broach and matching implant are supplied to the surgeon who uses them in a traditional manner to carry out total hip replacement. If it should be decided that cement should be used in the implantation, a smaller size implant would be manufactured, to allow for subsequent application of a uniform cement mantle.

The disclosed embodiment of the invention enables the supply of an assembled modular broach for surgical preparation of the femoral cavity to surgeon specified design determined by X-ray or CT scan templating, combined with preoperative manufacture of a matching hip prosthesis.

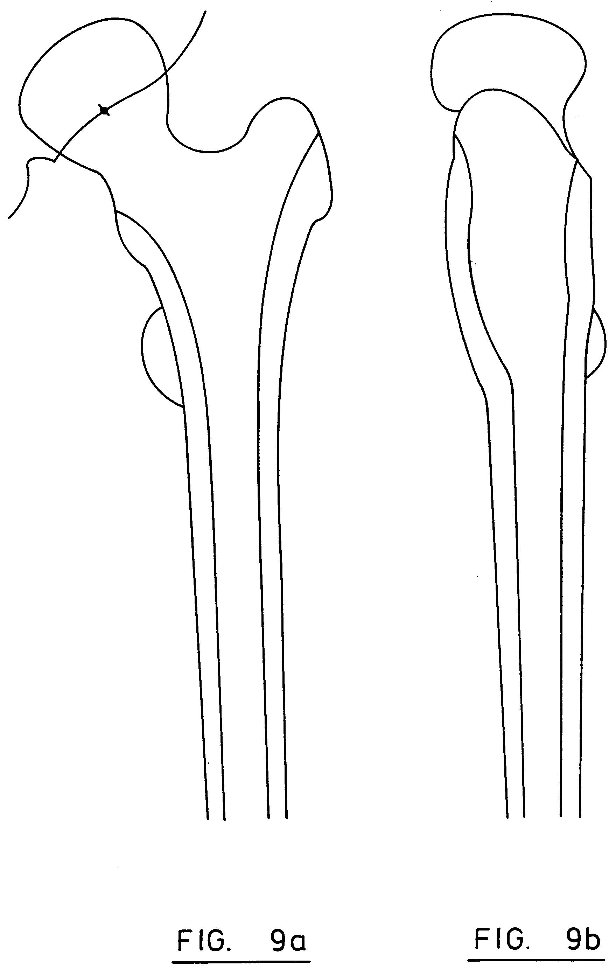

Referring now to Figures 9a and 9b to Figures 13, there will be described a further preferred example of method of manufacture of a prosthetic device in accordance with the invention, in which an alternative type of template is used.

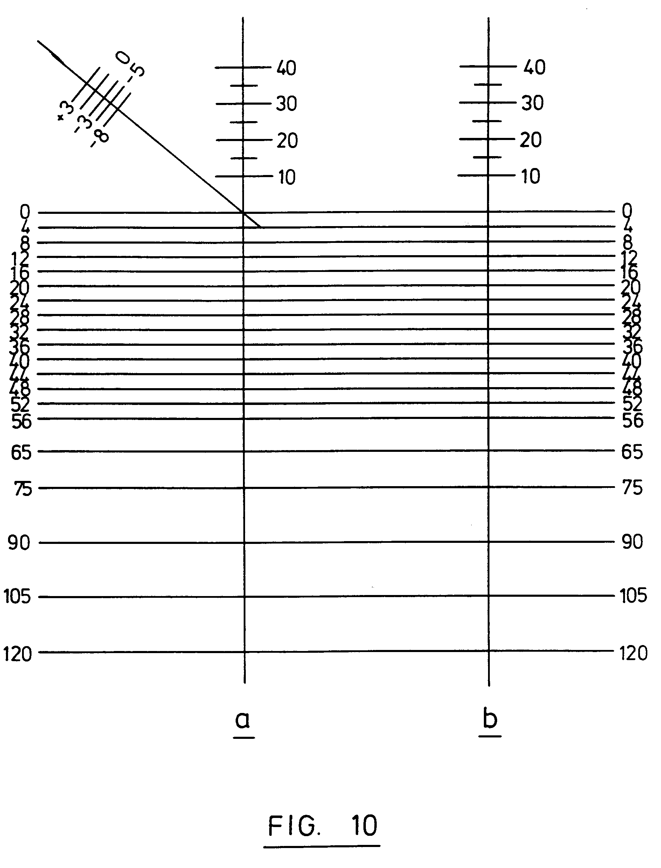

Figures 9a and 9b show anterior / posterior and lateral views of an x-ray image of a femoral bone cavity, and Figure 10 shows a template which can have the required outline shape of an implant marked thereon which is appropriate to the bone cavity, whereby to provide a template which defines the required shape of the implant which can be compared with the x-ray image views of the bone cavity, usually by superposing the template over the views shown in Figures 9a and 9b.

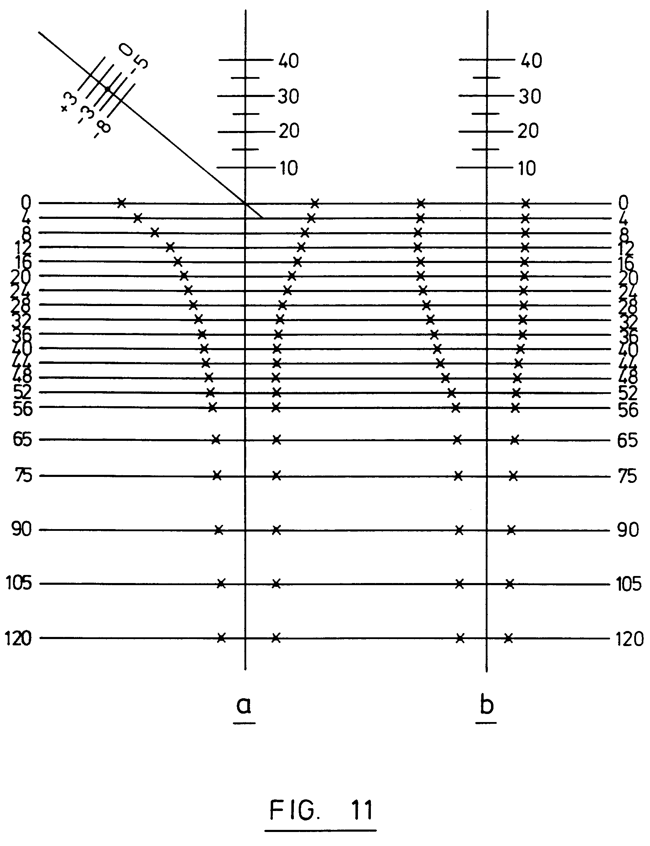

The reference lines a and b in Figure 10 correspond with the centre lines of the x-ray views 9a and 9b respectively, and once the reference lines shown in Figure 10 are lined-up with appropriate references on the x-ray images, the surgeon who will be carrying out the implant surgery can then apply a suggested outline profile for the implant as shown in Figure 11, again with references a and b corresponding with views 9a and 9b. This outline can be marked easily by the surgeon by making small crosses on the respective reference lines, and this is then compared with the respective x-ray images. The surgeon will use his expertise and experience, in his interpretation of the required outline in relation to the particular X-ray image of the bone cavity, to arrive at an optimum outline.

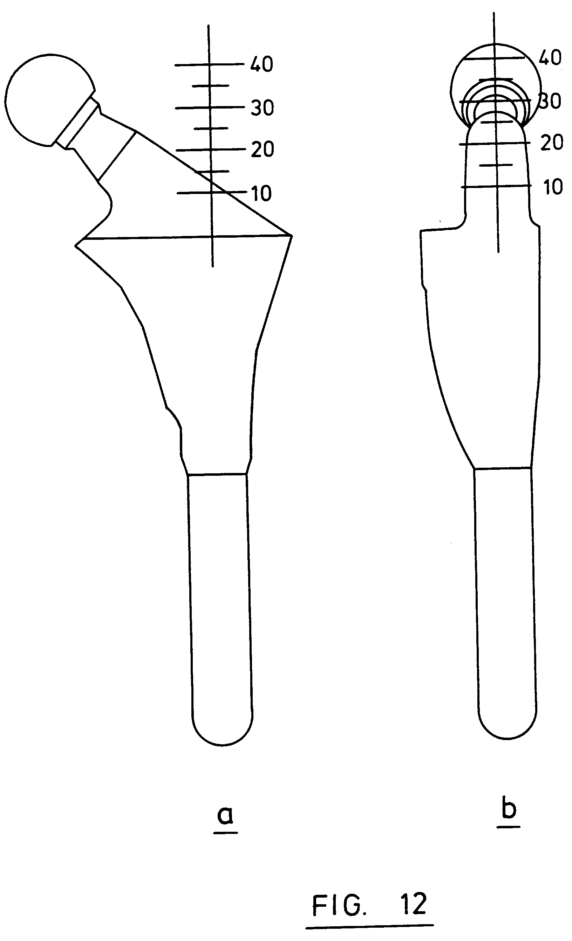

After the surgeon is satisfied with the visual comparison which he makes, or if necessary after providing an alternative implant shape for comparison with the image, then this information is passed back to the supplier / manufacturer of implants. The information on the template is then extracted and converted into digital form, from which is derived outline elevational views of the particular design of implant, as shown in Figure 12, in which references a and b designate the elevational views of the implant to suit the anterior / posterior and lateral views of Figures 9a and 9b respectively.

The views created for Figures 12a and 12b are derived from Figure 9a and 9b, and to a magnification which corresponds with the magnification factor of the originally formed X-ray images. The magnification factor will be presented on the X-ray images, and also on Figures 10 and 11. The views of Figures 12a and 12b will be on transparent sheet which can then be compared with the X-ray images.

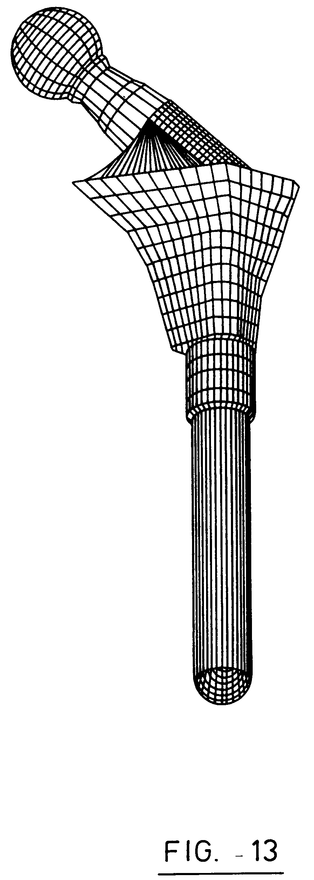

In addition, a computer generated graphics system is operated, with data derived from the template, to form a three dimensional representation of the required implant as shown in Figure 13.

The views of Figures 12 and 13 and X-ray templates are then conveyed to the surgeon for final approval, before the required implant is taken from stock, or manufactured to order.

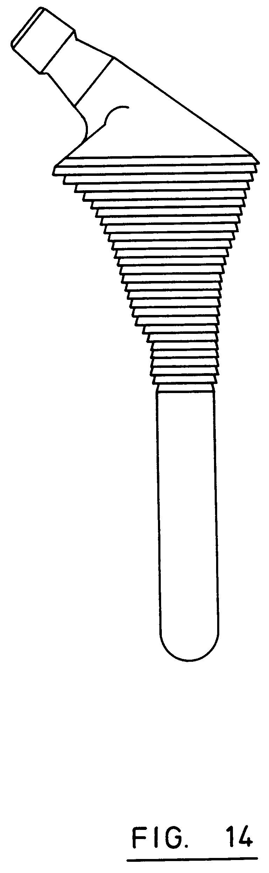

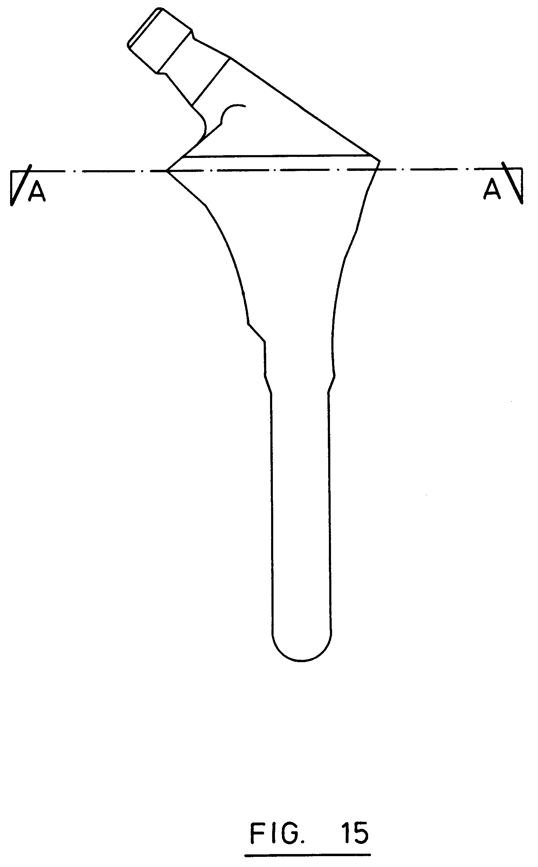

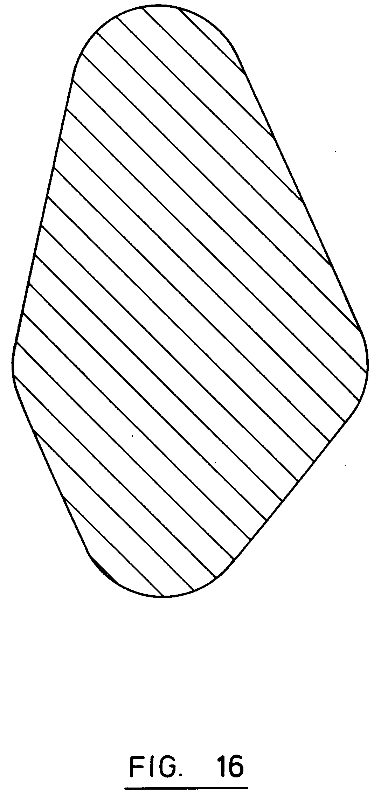

Figures 14 to 16 shows successive stages in the conversion of a two dimensional image to a suitable three dimensional implant form. To achieve this, the following preferred objectives should be borne in mind:
1. generate smooth points to give a shape that can be produced in the bone cavity;
2. determine the optimal distal diameter for circular cross-sections;
3. project the radius of the distal cross-section along the medial, lateral, anterior and posterior profiles;
4. tangentially join these with straight lines; or curved arcs could be used as an alternative.

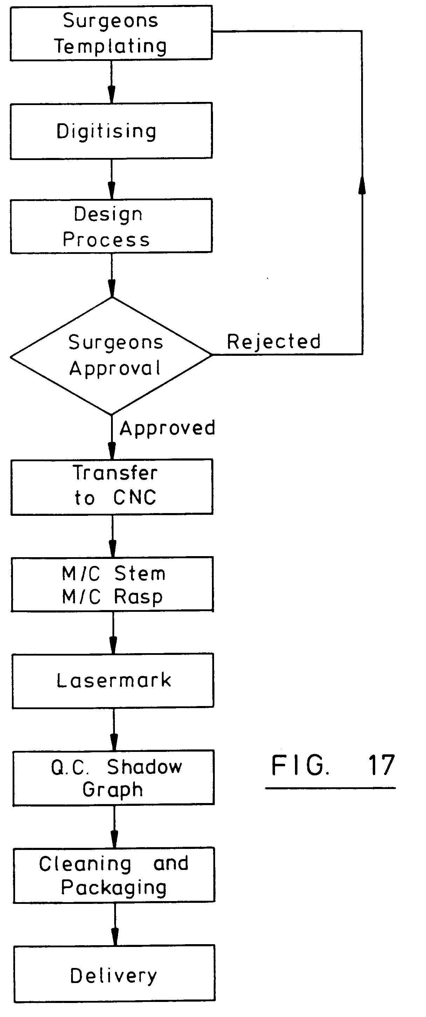

Figure 17 shows a flow diagram of successive stages which will be involved in the supply of custom designed implants to suit the requirements of the surgeon.

## Claims

1. A method of providing a prosthetic device for implantation in a bone cavity of a patient, which comprises:
(a) preparing an image of the bone cavity (11),
(b) providing a plurality of sets of templates (12, 13, 17, 18, 19) of a general shape appropriate to the implant to be fitted in the bone cavity, the templates of each set comprising a range of progressively varying shapes or sizes or both,
(c) making a selection from the said set of templates and comparing the selected template with the image to determine whether the template provides a suitable match with the profile of the cavity and, if necessary, making an alternative selection, and
(d) manufacturing an implant with a configuration corresponding to the selected template, or selecting an implant from a plurality of already-manufactured implants, with a configuration corresponding to the selected template,
characterised in that the templates form a two-dimensional model of the required implant which comprises a central core (12, 13), a proximo-medial segment (17), a proximo-lateral (18) segment and a proximo-anterior (19) segment.

2. A method as claimed in claim 1, in which the central core comprises a distal shaft portion (12) to be located over the main tubular portion of the cavity, and a proximal portion (13) to be located in the mouth or proximal end of the cavity.

3. A method as claimed in any one of claims 1 to 2, in which the image and the templates are compared by placing one of the image and template over the other.

4. A method as claimed in claim 3, in which one of the image and the template is transparent.

5. A method as claimed in any one of claims 1 to 4, in which the template has reference lines (15, 16) marked on it, and in which the method includes the step of aligning the reference lines with corresponding references on the image, and then marking the template manually on the template an appropriate outline for the implant.

6. A method as claimed in claim 5, which includes the step of digitising data marked on the template and deriving outline elevational views, or three-dimensional illustrations, or both, of the implant for review and approval prior to supply of the required design of implant.

## Patentansprüche

1. Verfahren zum Bereitstellen einer prothetischen Vorrichtung zur Implantation in einer Knochenhöhle eines Patienten, welches umfaßt:
(a) Herstellen eines Bildes der Knochenhöhle (11),
(b) Bereitstellen einer Vielzahl von Schablonensätzen (12, 13, 17, 18, 19) von allgemeiner, für das zu implantierende Implantat geeigneter Form, wobei die Schablonen jedes Satzes eine Bandbreite fortschreitend unterschiedlicher Formen oder Größen, oder beides, umfassen,
(c) Treffen einer Auswahl aus dem Satz von Schablonen und Vergleichen der ausgewählten Schablone mit dem Bild zur Festellung, ob die Schablone ein geeignetes, dem Profil der Höhle entsprechendes Gegenstück darstellt, und falls notwendig Treffen einer alternativen Auswahl,
(d) Herstellen eines Implantates mit einer Konfiguration entsprechend der ausgewählten Schablone, oder Auswählen eines Implantates mit einer Konfiguration entsprechend der ausgewählten Schablone aus einer Vielzahl von vorgefertigten Implantaten,
dadurch gekennzeichnet, daß die Schablonen ein zweidimensionales Modell des benötigten Implantates bilden, das einen zentralen Kern (12, 13), ein proximo-mediales Segment (17), ein proximo-laterales Segment (18) und ein proximoanteriores Segment (19) umaßt.

2. Verfahren nach Anspruch 1, in dem der zentrale Kern einen distalen Schaftteil (12), der auf den tubulären Hauptteil der Höhle gelegt wird, und einen proximalen Teil (13), der in die Mündung oder das proximale Ende der Höhle gelegt wird, umfaßt.

3. Verfahren nach einem der Ansprüche 1 oder 2, in dem das Bild mit der Schablone verglichen wird durch Auflegen des Bildes auf die Schablone bzw. der Schablone auf das Bild.

4. Verfahren nach Anspruch 3, in dem das Bild oder die Schablone transparent ist.

5. Verfahren nach einem der Ansprüche 1 bis 4, in dem auf der Schablone Bezugslinien (15, 16) markiert sind, und in dem das Verfahren als Verfahrensschritt das Ausrichten der Bezugslinien nach entsprechenden Bezügen auf dem Bild umfaßt, sowie anschließendes manuelles Markieren eines für das Implantat geeigneten Umrisses auf der Schablone.

6. Verfahren nach Anspruch 5, das den Schritt der Digitalisierung der auf der Schablone markierten Daten und Erstellung von Konturenaufrissen oder dreidimensionalen Abbildungen, oder von beidem, des Implantates anhand dieser Daten zur Prüfung und Bewilligung vor der Lieferung der gewünschten Ausführung des Implantates.

## Revendications

1. Procédé de fabrication d'une prothèse destinée à être implantée dans une cavité osseuse d'un patient, qui consiste à :
(a) préparer une image de la cavité osseuse (11),
(b) produire une pluralité d'ensembles de gabarits (12, 13, 17, 18, 19) de forme générale appropriée pour l'implant devant être inséré dans la cavité osseuse, les gabarits de chaque ensemble présentant une plage de diverses formes et/ou tailles variant progressivement,
(c) effectuer une sélection parmi ledit ensemble de gabarits et comparer le gabarit sélectionné à l'image pour déterminer si l'image correspond bien au profil de la cavité et, si nécessaire, effectuer une autre sélection, et
(d) fabriquer un implant dont la configuration correspond au gabarit sélectionné, ou sélectionner un implant parmi une pluralité d'implants déjà fabriqués, dont la configuration correspond au gabarit sélectionné,
caractérisé en ce que les gabarits forment un modèle bidimensionnel de l'implant requis qui comprend une partie centrale (12, 13), un segment proximo-médial (17), un segment proximo-latéral (18) et un segment proximo-antérieur (19).

2. Procédé selon la revendication 1, caractérisé en ce que la partie centrale comprend une partie distale formant tige (12) devant être placée sur la partie tubulaire centrale de la cavité, et une partie proximale (13) devant être placée dans l'embouchure ou l'extrémité proximale de la cavité.

3. Procédé selon l'une quelconque des revendications 1 à 2, caractérisé en ce que l'on compare l'image et les gabarits en les superposant les uns sur les autres.

4. Procédé selon la revendication 3, caractérisé en ce qu'un élément parmi l'image ou le gabarit est transparent.

5. Procédé selon l'une quelconque des revendications 1 à 4, caractérisé en ce que des lignes de repère (15, 16) sont tracées sur le gabarit, et en ce que le procédé comprend l'étape consistant à aligner les lignes de repère avec les repères correspondant sur l'image, puis à tracer manuellement sur le gabarit un contour correspondant à l'implant.

6. Procédé selon la revendication 5, caractérisé en ce qu'il comprend l'étape consistant à numériser les données indiquées sur le gabarit, à dériver des vues en élévation du contour, et/ou des illustrations tridimensionnelles de l'implant en vue de l'évaluation et l'approbation de ce dernier avant de fournir la conception requise de l'implant.
